# EUROPÄISCHE PATENTSCHRIFT

(11) **EP 1 440 056 B1**
(45) Veröffentlichungstag und Bekanntmachung des Hinweises auf die Patenterteilung: **23.12.2009**
(21) Anmeldenummer: 02785232.6
(22) Anmeldetag: 17.10.2002
(51) Int. Cl.: C07C 263/10

(54) **VERFAHREN ZUR HERSTELLUNG VON ISOCYANATEN**
METHOD FOR PRODUCING ISOCYANATES
PROCEDE DE PRODUCTION D'ISOCYANATES

(30) Priorität: 23.10.2001 DE 10152119
(43) Veröffentlichungstag der Anmeldung: 28.07.2004
(73) Patentinhaber: BASF SE, 67056 Ludwigshafen (DE)
(72) Erfinder: ROHDE, Thorsten, 68167 Mannheim (DE); STAMM, Armin, 55268 Nieder-Olm (DE); HENKELMANN, Jochem, 68165 Mannheim (DE)
(86) Internationale Anmeldenummer: PCT/EP2002/011625
(87) Internationale Veröffentlichungsnummer: WO 2003/035607

(56) Entgegenhaltungen:
- DE-A- 19 543 862
- DE-A- 19 928 688
- DE-A- 19 942 299
- US-A- 3 281 445
- US-A- 4 888 125
- "RÖMPP CHEMIE LEXICON, "SULFOLAN", 9. AUFLAGE" 1992, GEORG THIEME VERLAG , STUTTGART * Seite 4382 *
- "RÖMPP CHEMIE LEXIKON, "AMINE", 9. AUFLAGE" 1989, GEORG THIEME VERLAG , STUTTGART

## Beschreibung

Die vorliegende Erfindung betrifft ein Verfahren zur Herstellung von Isocyanaten durch Umsetzung der entsprechenden primären Amine mit Phosgen in einem inerten Lösungsmittel.

Isocyanate sind großtechnische Produkte mit einer Vielzahl von Anwendungen auf dem Gebiet der Polyurethan-Kunststoffe. Bestimmte Isocyanate finden aber auch Anwendung bei der Herstellung pharmazeutischer Wirkstoffe.

Die Synthese von Isocyanaten durch Umsetzung von Aminen mit Phosgen ist seit langen bekannt. Prinzipiell werden zwei Verfahren in der Literatur beschrieben, von denen das eine bei Normaldruck und das andere bei erhöhtem Druck durchgeführt wird. Die Phosgenierung unter erhöhtem Druck ist nachteilig, da sie zur Beherrschung des erhöhten Sicherheitsrisikos, der Freisetzung von Phosgen, einen stark erhöhten apparativ technischen Aufwand erfordert.

Für Sulfonylisocyanate ist aus US 3,371,114 und US 3,484,466 ein Herstellungsverfahren bei Normaldruck bekannt, bei dem eine Lösung eines Sulfonylamids und eines Isocyanats als Katalysator in einem inerten Lösungsmittel mit Phosgen umgesetzt wird. Dabei wird intermediär der entsprechende Sulfonylharnstoff gebildet, welcher mit Phosgen zum gewünschten Sulfonylisocyanat reagiert.

Alkyl- und Arylisocyanate werden üblicherweise nach dem beispielsweise in Ullmann's Encyclopedia of Industrial Chemistry, 6th edition, 2000 electronic release, Chapter "ISOCYANATES, ORGANIC - Production" beschriebenen Phosgenierungsverfahren in zwei Phasen bei Normaldruck aus den entsprechenden Aminen hergestellt. In der ersten Phase, der Kaltphosgenierung, wird das Amin mit einem Phosgentiberschuß in stark verdünnter Lösung und bei tiefen Temperaturen zum entsprechenden Carbaminsäurechlorid umgesetzt, aus dem in der zweiten Phase bei erhöhter Temperatur, der Heißphosgenierung, das Isocyanat entsteht. Aliphatische und cycloaliphatische primäre Amine sind dabei aufgrund ihrer gegenüber aromatischen Aminen erhöhten Basizität schlechter zu phosgenieren und führen zu einem vermehrten Anfall an Nebenprodukten. Nachteilig an diesen Verfahren ist neben der Phosgenierung in zwei Phasen, vor allem die Bildung einer intermediären Feststoffsuspension aus schwerlöslichem Carbaminsäurechlorid und Aminhydrochlorid, die wiederum eine erhöhte Verdünnung des Reaktionsmediums erforderlich macht, um Ablagerungen und Verstopfungen von Anlagenteilen zu verhindern. Aufgrund des vorhandenen Feststoffanfalls kann dieses Verfahren bei Normaldruck nicht kontinuierlich durchgeführt werden. Weiterhin entsteht ein symmetrisch N,N'-disubstituierter Harnstoff als Nebenprodukt, dessen Bildung nur um den Preis drastisch verringerter Raum/Zeit-Ausbeuten unterdrückt werden kann.

Aliphatisch und cycloaliphatische Amine werden häufig in Form ihrer Salze in die Kalt/Heißphosgenierung eingesetzt. Diese Salze sind jedoch im Reaktionsmedium schwer löslich, so daß zusätzliche Reaktionsstufen und sehr lange Reaktionszeiten erforderlich werden.

Weiterhin lehren US 3,440,268 und US 3,492,331 die Umsetzung primärer Amine mit Phosgen in Gegenwart von N,N-disubstituiertem Formamid, N-Alkyllactam, N,N-disubstituiertem N'-Aryl-formamidin und N,N,N',N'-tetrasubstituiertem N"-Aryl-guanidin als Katalysator. Aus H. Ulrich, Chemistry & Technology of Isocyanates, Wiley & Sons, 1996, Seiten 328 bis 330 ist die Umsetzung primärer Amine mit Phosgen in Gegenwart von tertiären Aminen, Tetramethylharnstoff und Carbonyldiimidazol als Katalysator sowie aus CS 262 295 in Gegenwart von N,N'-Diazabicyclo[2,2,2]oktan als Katalysator bekannt. Die genannten Verbindungen müssen zum Teil in äquimolaren Mengen eingesetzt werden und bilden unter den Reaktionsbedingungen schwer lösliche Salze in Form der Katalysator-Hydrochlorid-Addukte. Des weiteren bildet sich aus dem eingesetzten Amin und dem gebildeten Chlorwasserstoff schwerlösliches Amin-Hydrochlorid.

WO 01/17951 lehrt die Herstellung von Isocyanaten durch Phosgenierung der entsprechenden primären Amine in Gegenwart eines Monoisocyanats, welches vor Beginn der Umsetzung in einem inerten Lösungsmittel vorgelegt und mit Phosgen versetzt wird. Nachteilig an diesem Verfahren ist, dass die bevorzugt einzusetzenden niedermolekularen aliphatischen Isocyanate toxisch sind und hohe Sicherheitsvorkehnrngen erfordern.

DE 199 28 688 A beschreibt die Zugabe von cyclischen Sulfonsäureestern sowie von Sulfonen als Additive in der Polyaddition von Isocyanaten mit Alkoholen zur Verminderung der Bildung primärer Amine im Polyurethan-Schaumstoff. Gemäß der Lehre der DE-Offenlegungsschrift werden die Schwefelverbindungen vor der Polyaddition einer der beiden Einsatzstofflösungen zugefügt.

US 3,281,445 beschreibt die Zugabe von Schwefelverbindungen wie beispielsweise Sulfoxiden und Sulfonen zu Isocyanaten um die Verfärbung bei der Lagerung zu verzögern. Gemäß der Lehre des US-Patents werden die Schwefelverbindungen in das gereinigte Isocyanat eingebracht und vermischt. Bei Isocyanaten mit einem Schmelzpunkt oberhalb von Raumtemperatur werden diese zuvor durch Erwärmen aufgeschmolzen.

DE 199 42 299 A offenbart die Herstellung von Isocyanaten durch Umsetzung des entsprechenden primären Amins mit Phosgen in einem inerten Lösungsmittel.

US 4,888,125 lehrt die Herstellung von gegebenenfalls Methyl-substituierten Bis-(4-isocyanatophenoxy)-alkanen durch Umsetzung des entsprechenden primären Amins mit Phosgen in einem inerten Lösungsmittel.

Es bestand daher die Aufgabe, ein Verfahren zur Herstellung von Isocyanaten zu finden, welches die genannten Nachteile nicht mehr besitzt, sowohl kontinuierlich als auch diskontinuierlich durchführbar ist, keine oder nur eine unwesentliche Bildung schwerlöslicher Komponenten zeigt, ohne stark toxischem Katalysator durchführbar ist, die Umsetzung in nur einer Reaktionsstufe ermöglicht und auch unter milden Temperaturen und Drücken zu einem hohen Umsatz, einer hohen Selektivität und einer hohen Raum/Zeit-Ausbeute führt.

Demgemäß wurde ein Verfahren zur Herstellung von Isocyanaten durch Umsetzung der entsprechenden primären Amine mit Phosgen in einem inerten Lösungsmittel gefunden, das dadurch gekennzeichnet ist, dass man 0,01 bis 50 Mol-% eines Sulfoxids, eines Sulfons oder Gemische davon, bezogen auf die Gesamtmenge an primärem Amin und gebildetem Isocyanat in der Reaktionslösung, einsetzt.

Das beim erfindungsgemäßen Verfahren einsetzbare Sulfoxid besitzt die allgemeine Formel (I)

R¹-S(O)-R² (I)

sowie das beim erfindungsgemäßen Verfahren einsetzbare Sulfon die allgemeine Formel (II)

R¹-S (O)₂-R² (II),

in der die Reste R¹ bis R² jeweils unabhängig voneinander für einen Kohlenstoff enthaltenden organischen Rest stehen und gegebenenfalls auch miteinander verbunden sein können.

Unter einem Kohlenstoff enthaltenden organischen Rest ist ein unsubstituierter oder substituierter, aliphatischer, aromatischer oder araliphatischer Rest zu verstehen. Dieser kann ein oder mehrere Heteroatome, wie etwa Sauerstoff, Stickstoff, Schwefel oder Phosphor enthalten, beispielsweise -O-, -S-, -SO-, -SO₂-, -NR-, -CO-, -N=, -SiR₂-, -PR- und/oder -PR₂ und/oder durch eine oder mehrere funktionelle Gruppen, welche beispielsweise Sauerstoff, Stickstoff, Schwefel und/oder Halogen enthalten, substituiert sein, wie beispielsweise durch Fluor, Chlor, Brom, Iod und/oder eine Cyanogruppe (bei dem Rest R handelt es sich hierbei ebenfalls um einen Kohlenstoff enthaltenden organischen Rest). Bei dem Kohlenstoff enthaltenden organischen Rest kann es sich um einen einwertigen oder auch um einen zwei- oder dreiwertigen Rest handeln.

Als einwertige Reste stehen R¹ und R² jeweils unabhängig voneinander bevorzugt für
- einen unverzweigten oder verzweigten, acyclischen oder cyclischen, unsubstituierten oder substituierten Alkylrest mit 1 bis 30 aliphatischen Kohlenstoffatomen, bei dem eine oder mehrere der CH₂-Gruppen auch durch Heteroatome, wie -O- oder -S-, oder durch Heteroatom enthaltende Gruppen, wie -CO-, -SO-, -SO₂-, -NR- oder -SiR₂-, ersetzt sein können und bei dem ein oder mehrere der Wasserstoffatome durch Substituenten, wie beispielsweise Arylgruppen oder funktionelle Gruppen ersetzt sein können; oder für
- einen unsubstituierten oder substituierten aromatischen Rest mit 3 bis 30 Kohlenstoffatomen und einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl-, Arylgruppen oder funktionelle Gruppen ersetzt sein können.

Als zweiwertige Reste steht R¹ zusammen mit R² jeweils bevorzugt für
- einen unverzweigten oder verzweigten, acyclischen oder cyclischen, unsubstituierten oder substituierten C₄- bis C₂₀-Alkylenrest ("zweiwertiger Alkylrest") mit 4 bis 10 Atomen in der Alkylenkette, bei dem CH₂-Gruppen auch durch Heterogruppen, wie beispielsweise -CO-, -O-, -S-, -SO-, -SO₂-, -SiR₂- oder -NR- ersetzt sein können und bei dem ein oder mehrere der Wasserstoffatome durch Substituenten, wie beispielsweise Arylgruppen ersetzt sein können.

Besonders bevorzugt werden Sulfoxide (I) und/oder Sulfone (II) eingesetzt, deren Reste R¹ und R² jeweils unabhängig voneinander
- einen unverzweigten oder verzweigten C₁- bis C₂₀-Alkylrest, wie beispielsweise Methyl, Ethyl, 1-Propyl, 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl, 3-Methyl-3-pentyl oder. 2-Methoxy-2-propyl;
- einen unverzweigten oder verzweigten C₅- bis C₂₀-Cycloalkylrest, wie beispielsweise Cyclopentyl, Cyclohexyl oder Cyclooctyl; oder
- einen unsubstituierten oder mit einem oder mehreren C₁- bis C₄-Alkylresten substituierten C₆- bis C₂₀-Aryl- oder C₃- bis C₂₀-Heteroaryl-Rest, wie beispielsweise Phenyl, 2-Methylphenyl (o-Tolyl), 3-Methylphenyl (m-Tolyl), 4-Methylphenyl (p-Tolyl), 2,6-Dimethylphenyl, 2,4-Dimethylphenyl, 2,4,6-Trimethylphenyl, 2-Methoxyphenyl, 3-Methoxyphenyl, 4-Methoxyphenyl, 2-Pyridyl, 3-Pyridyl, 4-Pyridyl, 3-Pyridazinyl, 4-Pyridazinyl, 5-Pyridazinyl, 2-Pyrimidinyl, 4-pyrimidinyl, 5-Pyrimidinyl, 2-Pyrazinyl, 2-(1,3,5-Triazin)yl, 1-Naphthyl, 2-Naphthyl, 2-Chinolyl, 8-Chinolyl, 1-Isochinolyl oder 8-Isochinolyl;
   bedeuten.

Ganz besonders bevorzugt werden Sulfoxide (I) und/oder Sulfone (II) eingesetzt, deren Reste R¹ und R² jeweils unabhängig voneinander
- einen unverzweigten oder verzweigten C₁ bis C₁₀-Alkylrest;
- einen unverzweigten oder verzweigten C₅- bis C₁₀-Cycloalkylrest; oder
- einen unsubstituierten oder mit einem oder mehreren C₁- bis C₄-Alkylresten substituierten C₆- bis C₁₂-Aryl-Rest;
   bedeuten.

Insbesondere setzt man beim erfindungsgemäßen Verfahren Dimethylsuloxid oder Dimethylsulfon ein.

Das Sulfoxid, das Sulfon oder deren Gemisch werden in einer katalytischen Menge von 0,01 bis 50 Mol-%, bezogen auf die Gesamtmenge an primärem Amin und gebildetem Isocyanat in der Reaktionslösung, eingesetzt. Bei der Berechnung der Gesamtmenge an primärem Amin und gebildetem Isocyanat sind die Molmengen des noch nicht-umgesetzten Edukts (primäres Amin) und des bereits gebildeten Produkts (Isocyanat) sowie gegebenenfalls vorhandener Zwischenprodukte zu addieren. Beim erfindungsgemäßen Verfahren setzt man bevorzugt 0,01 bis 25 Mol-%, besonders bevorzugt 0,5 bis 20 Mol-% und ganz besonders bevorzugt 1 bis 15 Mol-% Sulfoxid, Sulfon oder deren Gemische, bezogen auf die Gesamtmenge an primärem Amin und gebildetem Isocyanat in der Reaktionslösung, ein.

Beim erfindungsgemäßen Verfahren legt man im Allgemeinen das Sulfoxid, das Sulfon oder deren Gemisch in einem inerten Lösungsmittel vor. Unter einem inerten Lösungsmittel sind Lösungsmittel zu verstehen, welche gegenüber dem eingesetzten primären Amin, dem Phosgen, dem gebildeten Isocyanat und dem verwendeten Sulfoxid oder Sulfon chemisch inert sind. "Chemisch inert" heißt, daß sich die Verdünnungsmittel unter den gewählten Bedingungen nicht mit den genannten Stoffen chemisch umsetzen. Bevorzugt setzt man aromatische oder aliphatische Kohlenwasserstoffe und besonders bevorzugt einfach oder mehrfach substituierte aromatische Kohlenwasserstoffe, wie beispielsweise Toluol, o-, m-, p-Xylol, Ethylbenzol. Chlorbenzol oder o-, m-, p-Dichlorbenzol ein. Ganz besonders bevorzugt sind o-, m- oder p-Xylol, Chlorbenzol, o-, m-, p-Dichlorbenzol oder deren Gemische.

Anschließend beginnt man im Allgemeinen mit der Einleitung von Phosgen. Es kann in flüssiger oder gasförmiger Form eingeleitet werden. In der Regel leitet man zunächst etwa 10 bis 50% der, bezogen auf das Reaktionsvolumen, theoretisch benötigten Menge an Phosgen ein.

In die mit Phosgen beladene Ausgangslösung von Sulfoxid, Sulfon oder deren Gemisch in inertem Lösungsmittel beginnt man nun, das primäre Amin zuzuführen. Phosgen wird dabei entsprechend dem Reaktionsfortgang und der Menge an zugeführten Amin weiter eingeleitet.

Die beim erfindungsgemäßen Verfahren einsetzbaren primären Amin besitzen die allgemeine Formel (II)

R³-NH₂ (II),

in der der Rest R³ für einen Kohlenstoff enthaltenden organischen Rest, wie zuvor definiert, steht.

Bevorzugt steht der Rest R³ für
- einen unverzweigten oder verzweigten, acyclischen oder cyclischen, unsubstituierten oder substituierten Alkylrest mit 1 bis 30 aliphatischen Kohlenstoffatomen, bei dem eine oder mehrere der CH₂-Gruppen auch durch Heteroatome, wie -O- oder -S-, oder durch Heteroatom enthaltende Gruppen, wie -CO-, -NR- oder -SiR₂-, ersetzt sein können und bei dem ein oder mehrere der Wasserstoffatome durch Substituenten, wie beispielsweise Arylgruppen oder funktionelle Gruppen, ausgenommen -OH, -SH, und -COOH Gruppen, ersetzt sein können; oder für
- einen unsubstituierten oder substituierten aromatischen Rest mit 3 bis 30 Kohlenstoffatomen und einem Ring oder zwei oder drei kondensierten Ringen, bei dem eines oder mehrere Ringatome durch Heteroatome, wie beispielsweise Stickstoff, substituiert sein können und bei dem ein oder mehrere der Wasserstoffatome durch Substituenten, wie beispielsweise Alkyl-, Arylgruppen oder funktionelle Gruppen, ausgenommen -OH, -SH und -COOH Gruppen, ersetzt sein können.

Insbesondere kann der Rest R³ eine oder mehrere weitere NH₂-Gruppen tragen, so dass als primäre Amine explizit auch Oligoamine mit zwei oder mehreren NH₂-Gruppen umfasst sind. Besonders bevorzugt setzt man als primäres Amin ein Monoamin mit einer NH₂-Gruppe oder ein Diamin mit zwei NH₂-Gruppen mit jeweils 1 bis 20 Kohlenstoffatomen ein.

Als Beispiele geeigneter acyclischer und gegebenenfalls substituierter Alkylreste Reste für R³ seien Methyl, Ethyl, 1-Propyl, 2-Propyl (sek.-Propyl), 1-Butyl, 2-Butyl (sek.-Butyl), 2-Methyl-1-propyl (iso-Butyl), 2-Methyl-2-propyl (tert.-Butyl), 1-Pentyl, 2-Pentyl, 3-Pentyl, 2-Methyl-2-butyl (tert.-Amyl), 1-Hexyl, 2-Hexyl, 3-Hexyl, 2-Methyl-2-pentyl. 3-Methyl-3-pentyl, 1-Octyl, 1-Decyl, 2-Aminoethyl, 3-Aminopropyl, 4-Aminobutyl, 5-Aminopentyl, 6-Aminohexyl, 8-Aminooctyl, Phenylmethyl, 1-Phenylethyl, 1-Phenylpropyl oder 1-Phenylbutyl genannt.

Als Beispiele cyclischer und gegebenenfalls substituierter Cycloalkylreste Reste für R³ seien Cyclopentyl, Cyclohexyl, Cyclooctyl oder 3-Aminomethyl-3,5,5-trimetyhlcyclohexyl genannt.

Als Beispiele aromatischer und heteroaromatischer und gegebenenfalls substituierter Reste für R³ seien Phenyl, o-, m-, p-Tolyl und Aminotolyle genannt.

Es sei betont, dass als primäre Amine auch enantiomerenreine, optisch aktive Verbindungen oder Gemische davon mit einem an der NH₂-Gruppe gebundenen optisch aktiven Kohlenstoffatom eingesetzt werden können. Das erfindungsgemäße Verfahren führt während der Reaktion und der Aufarbeitung vorteilhafterweise einem lediglich geringen Anteil an Racemisierung, der im Allgemeinen unter 2% liegt.

Ganz besonders bevorzugt setzt man bei erfindungsgemäßen Verfahren als primäres Amin 1,6-Diaminohexan, Cyclohexylamin, Isophorondiamin (3-Aminomethyl-3,5,5-trimetyhlcyclohexylamin), Anilin, Diaminotoluole, Diphenylmethan-4,4'-diamin, R-(+)- und S(-)-Phenylethylamin ein.

Das Molverhältnis zwischen der Gesamtmenge an zugeführtem Phosgen und den zu phosgenierenden primären Amin-Gruppen beträgt im Allgemeinen 1 bis 10, bevorzugt 1 bis 5 und besonders bevorzugt 1 bis 2, insbesondere 1,2 bis 1,8.

Die Menge an inertem Lösungsmittel beträgt im Allgemeinen 100 bis 2000 Gew.-% und bevorzugt 300 bis 1000 Gew.-%, bezogen auf die Gesamtmenge des vorliegenden primären Amins und des gebildeten Isocyanats.

Das erfindungsgemäße Verfahren führt man im Allgemeinen bei einer Temperatur von 20 bis 200°C, bevorzugt von 20 bis 150°C und besonders bevorzugt von 50 bis 100°C durch. Bei Durchführung des erfindungsgemäßen Verfahrens beträgt der Druck im Allgemeinen 0,05 bis 0,5 MPa abs und bevorzugt 0,08 bis 0,12 MPa abs.

Beim erfindungsgemäßen Verfahren legt man bevorzugt das Sulfoxid, das Sulfon oder dessen Gemisch in einem inerten Lösungsmittel vor, leitet darin Phosgen ein und führt in die mit Phosgen beladene Ausgangslösung das primäre Amin zu.

Nachdem die gewünschte Menge an Phosgen und Amin zugegeben wurde, wird die erhaltene Reaktionslösung in der Regel noch für eine gewisse Zeit, im Allgemeinen 30 Minuten bis 6 Stunden unter den Reaktionsbedingungen zur Nachreaktion belassen. Um das überschüssige Phosgen und dessen Reaktionsprodukte Kohlendioxid oder Chlorwasserstoff aus der Reaktionslösung zu entfernen beziehungsweise abzureichern, wird anschließend in der Regel unter intensiver Durchmischung Inertgas durchgeleitet ("Strippen").

Das erfindungsgemäße Verfahren kann prinzipiell kontinuierlich als auch diskontinuierlich durchgeführt werden, wobei eine kontinuierliche Verfahrensführung bevorzugt ist. Das Verfahren kann in einer beliebigen, für eine Umsetzung mit Phosgen geeigneten Anlage erfolgen. Als geeignete Reaktoren seien beispielsweise Rührkessel genannt.

Der Reaktionsaustrag wird im Allgemeinen mit den bekannten Methoden aufgearbeitet. Vorzugsweise wird das gewünschte Isocyanat durch fraktionierte Destillation isoliert.
Das als Katalysator eingesetzte Sulfoxid, Sulfon oder deren Gemisch wird dabei bevorzugt durch Destillation zurückgewonnen und bei einem kontinuierlichen Verfahren rückgeführt.

In einer allgemeinen Ausführungsform zur diskontinuierlichen Herstellung von Isocyanaten legt man das Sulfoxid, das Sulfon oder deren Gemisch in einem inerten Lösungsmittel im Reaktor, beispielsweise einem Rührkessel, unter Rühren vor und beläd die Lösung mit Phosgen. Das Reaktionssystem wird nun auf die gewünschte Temperatur gebracht und mit der Zufuhr des Amins begonnen. Phosgen wird dabei entsprechend dem Reaktionsfortgang und der Menge an zugeführten Amin weiter eingeleitet. Nachdem die gewünschte Menge an Phosgen zugeführt wurde, wird die Reaktionslösung noch einige Zeit bei der eingestellten Temperatur unter fortgesetztem Rühren zur Nachreaktion belassen. Bei der Nachreaktion setzt sich noch in der Reaktionslösung befindliches Phosgen mit restlichem Amin um. Um das überschüssige Phosgen und dessen Reaktionsprodukte Kohlendioxid oder Chlorwasserstoff aus der Reaktionslösung zu entfernen beziehungsweise abzureichern, ist es möglich, unter intensiver Durchmischung Inertgas durchzuleiten ("Strippen"). Die erhaltene Reaktionslösung wird nun der Aufarbeitung zugeführt. Im Allgemeinen erfolgt die Aufarbeitung destillativ, gegebenenfalls unter Vakuum. Bei höhersiedenden Isocyanaten sind auch andere Reinigungsverfahren, wie beispielsweise Kristallisation möglich.

In einer allgemeinen Ausführungsform zur kontinuierlichen Herstellung von Isocyanaten legt man das Sulfoxid, das Sulfon oder deren Gemisch in einem inerten Lösungsmittel im Reaktor, beispielsweise einem Rührkessel, unter Rühren vor und beläd die Lösung mit Phosgen. Das Reaktionssystem wird nun auf die gewünschte Temperatur gebracht und mit der kontinuierlichen Zufuhr des Amins begonnen. Phosgen wird dabei entsprechend dem Reaktionsfortgang und der Menge an zugeführten Amin kontinuierlich eingeleitet. Nachdem sich der Reaktorinhalt weitgehend zum Isocyanat umgesetzt hat, gleicht man die Mengen an Amin und Phosgen derart an, daß beide im Wesentlichen in stöchiometrisch erforderlichem Verhältnis zugeführt werden. Eine der zugefahrenen Menge entsprechende Menge des Reaktionsvolumens wird dem Reaktionsapparat, beispielsweise über eine Standhaltung oder einem Überlauf, entnommen. Die entnommene Reaktionslösung wird in einem nachgeschalteten Behälter, beispielsweise einem Rührkessel, zur Nachreaktion aufgefangen. Nachdem auch der nachgeschaltete Behälter durch den Reaktionsaustrag gefüllt wurde, wird der Überlauf gegebenenfalls von den Koppelprodukten Kohlendioxid und Chlorwasserstoff wie oben beschrieben durch Strippen befreit und der Aufarbeitung zugeführt. Die Aufarbeitung kann beispielsweise destillativ durchgeführt werden.

Das erfindungsgemäße Verfahren ermöglicht die kontinuierliche oder diskontinuierliche Herstellung von Isocyanaten durch Phosgenierung von primären Aminen. Es ermöglicht gegenüber den bekannten, katalysatorfreien Verfahren die Durchführung der eigentlichen Umsetzung in nur einer einzigen Reaktionsstufe unter milden Temperaturen und Drücken, wobei ebenfalls ein hoher Umsatz an primärem Amin, eine hohe Selektivität und eine hohe Raum/ZeitAusbeute an Isocyanat erreicht wird. Gegenüber den bekannten, katalysatorfreien Verfahren und den bekannten Verfahren in Gegenwart eines Katalysator weist das erfindungsgemäße Verfahren keine oder nur eine unwesentliche Tendenz zur Bildung schwerlöslicher Komponenten auf. Das Risiko zur Ablagerung an und Verstopfung von Anlagenteilen wird dadurch deutlich minimiert, was beim Umgang mit dem toxischen Phosgen einen entscheidenden Sicherheitsgewinn darstellt. Ferner wird duch die ausbleibende oder nur unwesentliche Bildung schwerlöslicher Komponenten die Durchführung eines kontinuierlichen Verfahrens erst ermöglicht und signifikante Vorteile bei der anschließenden Aufarbeitung erzielt.

Die beim erfindungsgemäßen Verfahren einzusetzenden Sulfoxide und Sulfone sind in der Regel nicht oder nur minder toxisch, was vor allem einen Vorteil gegenüber dem in WO 01/17951 beschriebenen Einsatz eines aliphatischen Monoisocyanats darstellt.

### Beispiele

### Versuchsanordnung

Die Versuchsanordnung umfasste ein 1 L Glasgefäß mit einem Rührer, einer Thermostatierung, einem Einleitungsrohr für das gasförmige Phosgen und einer zweiteiligen Kühlerkaskade. Die zweiteilige Kühlerkaskade umfasst einen Intensivkühler, welcher auf -10°C temperiert wurde und einen Kohlensäurekühler, welcher auf -78°C temperiert wurde. Die Versuche wurden bei Atmosphärendruck durchgeführt.

### Vergleichsbeispiel 1

500 g Chlorbenzol wurden im Glasgefäß vorgelegt und bei Raumtemperatur 40 g Phosgen eingegast. Anschließend wurde der Reaktionsansatz auf 77°C erhitzt, wobei sich ein kräftiger Phosgenrückfluß einstellte. Unter intensivem Rühren nun wurden bei 77 bis 80°C innerhalb von 3 Stunden insgesamt 99,2 g Cyclohexylamin (1 Mol), gelöst in 200 g Chlorbenzol, und gleichzeitig weitere 92 g Phosgen zugeführt. Nach beendeter Zufuhr wurde das System zur Nachreaktion eine weitere Stunde ohne Phosgenzufuhr bei 77 bis 80°C belassen und anschließend das restliche, nicht-umgesetzte Phosgen mit Stickstoff bei 50°C herausgestrippt. Bei dem erhaltenen Reaktionsgemisch handelte es sich um eine Suspension, von der der enthaltene Feststoff durch Filtration abgetrennt wurde. Es konnten dabei 15 g Feststoff, bei dem es sich laut IR-spektroskopischer Analyse hauptsächlich um Aminhydrochlorid handelte, isoliert werden. Der filtrierte Rohaustrag wurde destillativ aufgearbeitet. Nach Abtrennung des Lösungsmittels und fraktionierter Destillation wurden 88,8 g Cyclohexylisocyanat (0,710 Mol) isoliert. Dies entspricht 71,0% der theoretischen Ausbeute.

### Beispiel 2 (erfindungsgemäß)

500 g Chlorbenzol und 2 g Dimethylsulfoxid (0,025 Mol) wurden im Glasgefäß vorgelegt und bei Raumtemperatur 35 g Phosgen eingegast. Anschließend wurde der Reaktionsansatz auf 78°C erhitzt, wobei sich ein kräftiger Phosgenrückfluß einstellte. Unter intensivem Rühren nun wurden bei 78 bis 82°C innerhalb von 3 Stunden insgesamt 99,2 g Cyclohexylamin (1 Mol), gelöst in 200 g Chlorbenzol, und gleichzeitig weitere 113 g Phosgen zugeführt. Nach beendeter Zufuhr wurde das System zur Nachreaktion eine weitere Stunde ohne Phosgenzufuhr bei 78 bis 82°C belassen und anschließend das restliche, nicht-umgesetzte Phosgen mit Stickstoff bei 40°C herausgestrippt. Bei dem erhaltenen Reaktionsgemisch handelte es sich um eine dünnflüssige Suspension, von der der enthaltene Feststoff durch Filtration abgetrennt wurde. Es konnten dabei 2,2 g Feststoff isoliert werden. Der filtrierte Rohaustrag wurde destillativ aufgearbeitet. Nach Abtrennung des Lösungsmittels und fraktionierter Destillation wurden 79,7 g Cyclohexylisocyanat (0,631 Mol) isoliert. Dies entspricht 63,1% der theoretischen Ausbeute.

### Beispiel 3 (erfindungsgemäß)

500 g Chlorbenzol und 2,4 g Dimethylsulfon (0,025 Mol) wurden im Glasgefäß vorgelegt und bei Raumtemperatur 30 g Phosgen eingegast. Anschließend wurde der Reaktionsansatz auf 78°C erhitzt, wobei sich ein kräftiger Phosgenrückfluß einstellte. Unter intensivem Rühren nun wurden bei 78 bis 80°C innerhalb von 3 Stunden insgesamt 99,2 g Cyclohexylamin (1 Mol), gelöst in 200 g Chlorbenzol, und gleichzeitig weitere 105 g Phosgen zugeführt. Nach beendeter Zufuhr wurde das System zur Nachreaktion eine weitere Stunde ohne Phosgenzufuhr bei 78 bis 80°C belassen und anschließend das restliche, nicht-umgesetzte Phosgen mit Stickstoff bei 50°C herausgestrippt. Bei dem erhaltenen Reaktionsgemisch handelte es sich um eine Suspension, von der der enthaltene Feststoff durch Filtration abgetrennt wurde. Es konnten dabei 7,8 g Feststoff isoliert werden. Der filtrierte Rohaustrag wurde destillativ aufgearbeitet. Nach Abtrennung des Lösungsmittels und fraktionierter Destillation wurden 86,8 g Cyclohexylisocyanat (0,694 Mol) isoliert. Dies entspricht 69,4% der theoretischen Ausbeute.

Das erfindungsgemäße Beispiel 2 unter Einsatz von Dimethylsulfoxid zeigt einen um Faktor 7 und das erfindungsgemäße Beispiel 3 unter Einsatz von Dimethylsulfon einen um Faktor 2 niedrigeren Feststoffanteil als das Vergleichsbeispiel 1 ohne Einsatz eines Katalysators. Es ist somit wesentlich weniger Feststoff abzutrennen.

Durch den niedrigeren Feststoffanteil läßt sich das erfindungsgemäße Verfahren wesentlich einfacher, sicherer und problemloser durchführen. Insbesondere wird das Risiko zur Ablagerung an und Verstopfung von Anlagenteilen deutlich minimiert, was beim Umgang mit dem toxischen Phosgen einen entscheidenden Sicherheitsgewinn darstellt und die Durchführung eines kontinuierlichen Verfahrens ermöglicht.

Durch die Möglichkeit, ein kontinuierliches Verfahren durchführen zu können, wird gegenüber den bisher, aufgrund des Feststoffanfalls erforderlichen diskontinuierlichen Verfahren eine deutliche Zunahme der Raum/Zeit-Ausbeute erzielt. Nicht-umgesetztes Amin kann dem Reaktionsapparat wieder rückgeführt werden, so dass auch die erzielbare Ausbeute höher liegt als bei den bekannten Verfahren.

## Patentansprüche

1. Verfahren zur Herstellung von Isocyanaten durch Umsetzung der entsprechenden primären Amine mit Phosgen in einem inerten Lösungsmittel, **dadurch gekennzeichnet, dass** man 0,01 bis 50 Mol-°% eines Sulfoxids, eines Sulfons oder Gemische davon, bezogen auf die Gesamtmenge an primärem Amin und gebildetem Isocyanat in der Reaktionslösung, einsetzt.

2. Verfahren nach Anspruch 1, **dadurch gekennzeichnet, dass** man 0,01 bis 25 Mol-% des Sulfoxids, des Sulfons oder deren Gemische, bezogen auf die Gesamtmenge an primärem Amin und gebildetem Isocyanat in der Reaktionslösung, einsetzt.

3. Verfahren nach den Ansprüchen 1 bis 2, **dadurch gekennzeichnet, dass** man als Sulfoxid ein Sulfoxid der allgemeinen Formel (I)
R¹-S(O)-R² (I)
und/oder als Sulfon ein Sulfon der allgemeinen Formel (II)
R¹-S(O)₂-R² (II),
in der die Reste R¹ und R² jeweils unabhängig voneinander einen unverzweigten oder verzweigten C₁- bis C₁₀-Alkylrest, einen unverzweigten oder verzweigten C₅-bis C₁₀-Cycloalkylrest oder einen unsubstituierten oder mit einem oder mehreren C₁- bis C₄-Alkylresten substituierten C₆- bis C₁₂-Aryl-Rest bedeuten, einsetzt.

4. Verfahren nach Anspruch 3, **dadurch gekennzeichnet, dass** man Dimethylsulfoxid oder Dimethylsulfon einsetzt.

5. Verfahren nach den Ansprüchen 1 bis 4, **dadurch gekennzeichnet, dass** man als primäres Amin ein Monoamin mit einer NH₂-Gruppe oder ein Diamin mit zwei NH₂-Gruppen mit jeweils 1 bis 20 Kohlenstoffatomen einsetzt.

6. Verfahren nach Anspruch 5, **dadurch gekennzeichnet, dass** man als primäres Amin 1,6-Diaminohexan, Cyclohexylamin, Isophorondiamin, Anilin, Diaminotoluole, Diphenylmethan-4,4'-diamin, R-(+)- und S(-)-Phenylethylamin einsetzt.

7. Verfahren nach den Ansprüchen 1 bis 6, **dadurch gekennzeichnet, dass** man die Umsetzung bei einer Temperatur von 20 bis 200°C und einem Druck von 0,05 bis 0,5 MPa abs durchführt.

8. Verfahren nach den Ansprüchen 1 bis 7, **dadurch gekennzeichnet, dass** man das inerte Lösungsmittel in einer Menge von 100 bis 2000 Gew.-%, bezogen auf die Gesamtmenge des vorliegenden primären Amins und des gebildeten Isocyanats, einsetzt.

9. Verfahren nach den Ansprüchen 1 bis 8, **dadurch gekennzeichnet, dass** man als inertes Lösungsmittel o-, m- oder p-Xylol, Chlorbenzol, o-, m-, p-Dichlorbenzol oder deren Gemische einsetzt.

10. Verfahren nach den Ansprüchen 1 bis 9, **dadurch gekennzeichnet, dass** man das Sulfoxid, das Sulfon oder dessen Gemisch in einem inerten Lösungsmittel vorlegt, darin Phosgen einleitet und in die mit Phosgen beladene Ausgangslösung das primäre Amin zuführt.

## Claims

1. A process for preparing isocyanates by reacting the corresponding primary amines with phosgene in an inert solvent, wherein from 0.01 to 50 mol% of a sulfoxide, a sulfone or a mixture thereof, based on the total amount of primary amine and isocyanate formed in the reaction solution, is used.

2. The process according to claim 1, wherein from 0.01 to 25 mol% of the sulfoxide, the sulfone or the mixture thereof, based on the total amount of primary amine and isocyanate formed in the reaction solution, is used.

3. The process according to claim 1 or 2, wherein a sulfoxide of the formula (I)
R¹- S(O)-R² (I)
is used as sulfoxide and/or a sulfone of the formula (II)
R¹-S(O)₂-R² (II),
is used as sulfone, where the radicals R¹ and R² are each, independently of one another, an unbranched or branched C₁-C₁₀-alkyl radical, an unbranched or branched C₅-C₁₀-cycloalkyl radical or a C₆-C₁₂-aryl radical which may be unsubstituted or substituted by one or more C₁-C₄-alkyl radicals.

4. The process according to claim 3, wherein dimethyl sulfoxide or dimethyl sulfone is used.

5. The process according to any of claims 1 to 4, wherein the primary amine used is a monoamine having one NH₂ group or a diamine having two NH₂ groups, each having from 1 to 20 carbon atoms.

6. The process according to claim 5, wherein the primary amine used is 1,6-diaminohexane, cyclohexylamine, isophoronediamine, aniline, a diaminotoluene, diphenylmethane-4,4'-diamine, R-(+)- or S(-)-phenylethylamine.

7. The process according to any of claims 1 to 6, wherein the reaction is carried out at from 20 to 200°C and a pressure of from 0.05 to 0.5 MPa abs.

8. The process according to any of claims 1 to 7, wherein the inert solvent is used in an amount of from 100 to 2000% by weight, based on the total amount of primary amine present and isocyanate formed.

9. The process according to any of claims 1 to 8, wherein the inert solvent used is o-, m- or p-xylene, chlorobenzene, o-, m-, p-dichlorobenzene or a mixture thereof.

10. The process according to any of claims 1 to 9, wherein the sulfoxide, the sulfone or the mixture thereof and an inert solvent are initially charged, phosgene is introduced into the mixture and the primary amine is fed into the phosgeneladen starting solution.

## Revendications

1. Procédé pour la préparation d'isocyanates par transformation des amines primaires correspondantes avec du phosgène dans un solvant inerte, **caractérisé en ce qu'**on utilise 0,01 à 50% en mole d'un sulfoxyde, d'une sulfone ou de leurs mélanges, par rapport à la quantité totale d'amine primaire et d'isocyanate formé, dans la solution réactionnelle.

2. Procédé selon la revendication 1, **caractérisé en ce qu'**on utilise 0,01 à 25% en mole du sulfoxyde, de la sulfone ou de leurs mélanges, par rapport à la quantité totale d'amine primaire et d'isocyanate formé, dans la solution réactionnelle.

3. Procédé selon les revendications 1 à 2,
**caractérisé en ce qu'**on utilise comme sulfoxyde un sulfoxyde de formule générale (I)
R¹-S(O)-R² (I)
et/ou comme sulfone une sulfone de formule générale (II)
R¹-S(O)₂R² (II),
dans lesquelles les radicaux R¹ et R² signifient, à chaque fois indépendamment l'un de l'autre, un radical C₁-C₁₀-alkyle non ramifié ou ramifié, un radical C₅-C₁₀-cycloalkyle non ramifié ou ramifié ou un radical C₆-C₁₂-aryle non substitué ou substitué par un ou plusieurs radicaux C₁-C₄-alkyle.

4. Procédé selon la revendication 3, **caractérisé en ce qu'**on utilise du diméthylsulfoxyde ou de la diméthylsulfone.

5. Procédé selon les revendications 1 à 4,
**caractérisé en ce qu'**on utilise comme amine primaire une monoamine comprenant un groupe NH₂ ou une diamine comprenant deux groupes NH₂ avec à chaque fois 1 à 20 atomes de carbone.

6. Procédé selon la revendication 5, **caractérisé en ce qu'**on utilise comme amine primaire du 1,6-diaminohexane, de la cyclohexylamine, de l'isophoronediamine, de l'aniline, du diaminotoluène, de la diphénylméthane-4,4'-diamine, de la R-(+)-phényléthylamine et S(-)-phényléthylamine.

7. Procédé selon les revendications 1 à 6,
**caractérisé en ce qu'**on réalise la transformation à une température de 20 à 200°C et une pression de 0,05 à 0,5 MPa abs.

8. Procédé selon les revendications 1 à 7,
**caractérisé en ce qu'**on utilise le solvant inerte en une quantité de 100 à 2000% en poids, par rapport à la quantité totale de l'amine primaire concernée et de l'isocyanate formé.

9. Procédé selon les revendications 1 à 8,
**caractérisé en ce qu'**on utilise comme solvant inerte de l'o-xylène, du m-xylène ou du p-xylène, du chlorobenzène, de l'o-dichlorobenzène, du m-dichlorobenzène, du p-dichlorobenzène ou leurs mélanges.

10. Procédé selon les revendications 1 à 9,
**caractérisé en ce qu'**on dispose au préalable le sulfoxyde, la sulfone ou leur mélange dans un solvant inerte, on y introduit le phosgène et on introduit l'amine primaire dans la solution de départ chargée de phosgène.
